# EUROPEAN PATENT APPLICATION

(11) **EP 2 583 697 A1**
(43) Date of publication of application: **24.04.2013**
(21) Application number: 11186214.0
(22) Date of filing: 21.10.2011
(51) Int. Cl.: A61L 15/22, A61L 15/42

(54) **Absorbent core**

(71) Applicant: The Procter & Gamble Company, Cincinnati, OH 45202 (US)
(72) Inventor: Carlucci, Giovanni, 66100 CHIETI (IT)
(74) Representative: Briatore, Andrea

(57) **Abstract**

Absorbent core for disposable absorbent articles comprising absorbent gelling material and expandable microspheres.

## Description

### FIELD OF THE INVENTION

The present invention relates to an absorbent core for absorbent articles, for example sanitary napkins and the like.

### BACKGROUND OF THE INVENTION

Absorbent articles for absorption of body fluids such as menses or blood or vaginal discharges or urine are well known in the art, and comprise for example feminine hygiene articles such as sanitary napkins, panty liners, tampons, interlabial devices, as well as wound dressings, baby diapers, article for adult incontinence, and the like. When considering for example sanitary napkins, these articles typically comprise a liquid-pervious topsheet as wearer-facing layer, a backsheet as garment-facing layer and an absorbent core between topsheet and backsheet. The body fluids are acquired through the topsheet and subsequently stored in the absorbent core. The backsheet typically prevents the absorbed fluids from wetting the wearer's garment.

An absorbent core can typically comprise one or more fibrous absorbent material, which in turn can comprise natural fibres, such as for example cellulose fibres, typically wood pulp fibres, synthetic fibres, or combinations thereof.

Absorbent articles can further comprise, typically in the absorbent core, superabsorbent materials, such as absorbent gelling materials (AGM), usually in finely dispersed form, e.g. typically in particulate form, in order to improve their absorption and retention characteristics. Superabsorbent materials for use in absorbent articles typically comprise water-insoluble, water-swellable, hydrogel-forming crosslinked absorbent polymers which are capable of absorbing large quantities of liquids and of retaining such absorbed liquids under moderate pressure.

Improved comfort for the user has been since long recognized as a need for this category of products. Thin disposable absorbent articles, typically featuring a thickness of less than 5 mm, are for example appreciated since they can be more comfortable and discreet.

For thin structures absorbent gelling materials are incorporated in absorbent articles, typically in the core structure, in localized and more concentrated arrangement with relatively high amounts, e.g. between web layers.

Improvements in thinness in disposable absorbent articles sometimes have led to compromise with the level of softness in dry condition and of protection in wet condition of said articles.

A thinner article, that may be preferred in terms of conformability and discreetness, can in fact lead to roughness in use when still dry due to the feeling by the user's body of the particulate form of the absorbing gelling materials. Also, a thinner absorbent core with relatively high amounts of absorbent gelling material can still be improved in its absorption and retention capacity towards body fluids. In particular, sudden surges are particularly difficult to be effectively absorbed and retained into thin absorbent cores containing superabsorbent materials in major amounts since such thin cores may not have enough free space for the expansion of absorbent gelling materials upon fluid absorption.

It is believed that this adverse effect can also be enhanced by the fact that the absorbent gelling material, usually in form of particles, can be provided in relatively high amount and concentration, typically with particles in close contact to one another, at least in some regions of the absorbent core, which upon absorption of fluid and subsequent swelling might involve a reduced permeability of the layer of absorbent material to further fluid releases. This may translate into a slower fluid uptake into the superabsorbent material, and in turn into the absorbent structure comprising the superabsorbent material, which can result in a lower overall absorption and retention capacity.

The present invention provides significant improvements in the above areas by the incorporation of a material based on microspheres encapsulating a gas in the absorbent core structure for an absorbent article, particularly for absorption of menses or blood or vaginal discharges, which comprises the absorbent gelling material.

### SUMMARY OF THE INVENTION

The present invention addresses the above needs by providing a layered absorbent core, which comprises a substrate layer, a layer of absorbent polymer material comprising a non water soluble thermoplastic material. The layer of absorbent polymer material further comprises microspheres.

### BRIEF DESCRIPTION OF THE DRAWINGS

Figure 1 is a plan view of a sanitary napkin showing an absorbent core according to an embodiment of the present invention, with portions of some constituent elements cut out in order to show underlying elements.
Figure 2 is a schematic cross section of the sanitary napkin of Figure 1 taken in the transverse axis A-A'.
Figure 3 shows a schematic cross section of an absorbent core according to one embodiment of the present invention.
Figure 4 shows a schematic cross section of an absorbent core according to another embodiment of the present invention.
Figure 5 shows a perspective view of an exemplary absorbent core according to the present invention.

### DETAILED DESCRIPTION OF THE INVENTION

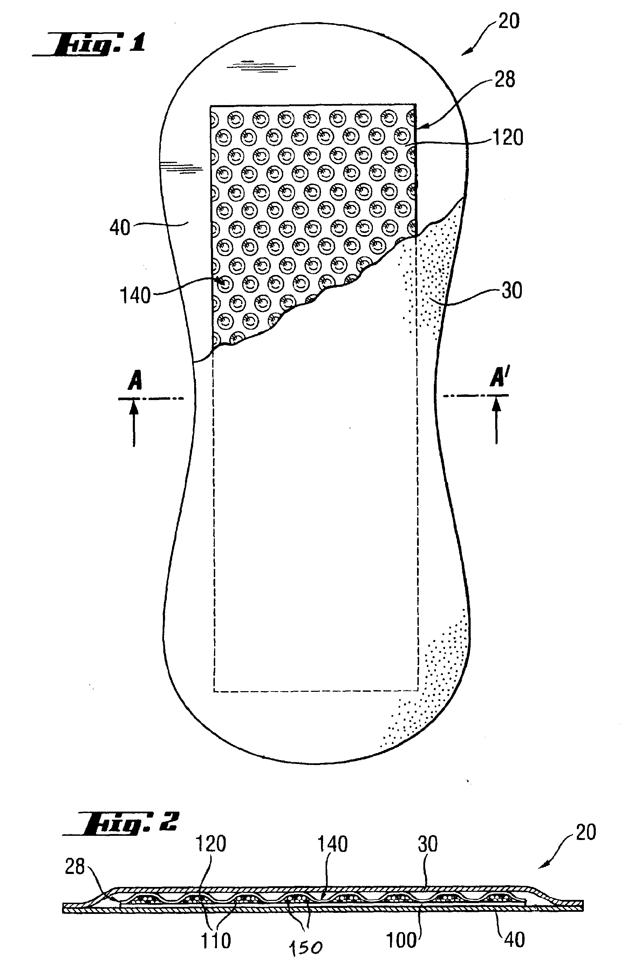

The present invention relates to an absorbent core for absorbent articles such as sanitary napkins, panty liners, tampons, interlabial devices, wound dressings, baby diapers, adult incontinence articles, and the like, which are intended for the absorption of body fluids, such as menses or blood or vaginal discharges or urine. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). The absorbent core of the present invention will be herein described in the context of a typical absorbent article, such as, for example, a sanitary napkin 20 as illustrated in Figure 1. Typically, such articles as shown in Figure 1 can comprise the elements of a liquid pervious topsheet 30, a backsheet 40 and an absorbent core 28 intermediate said topsheet 30 and said backsheet 40.

In the following description of the invention, the surface of the article, or of each element thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

### Topsheet

According to the present invention, the absorbent article can comprise a liquid pervious topsheet. The topsheet suitable for use herein can comprise wovens, non-wovens, and/or three-dimensional webs of a liquid impermeable polymeric film comprising liquid permeable apertures. In Figure 1 the topsheet is indicated with reference numeral 30. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

### Absorbent Core

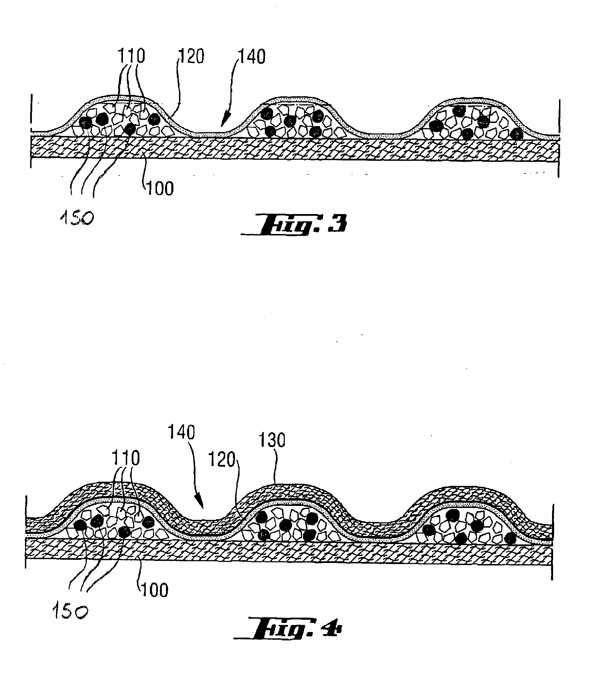

According to the present invention, the absorbent element is typically a thin, high-capacity absorbent core. Typical arrangements of the invention comprise an absorbent core which contains absorbent polymer material as absorbent material, and typically as the main absorbent material. Typically, for example with reference to Figures 2 and 3, the absorbent core 28 is a layered structure comprising a substrate 100, a layer of absorbent polymer material 110 also comprising a non water soluble thermoplastic material 120 and microspheres 150.

Typically the absorbent polymer material 110 for the absorbent core 28 according to the present invention can comprise absorbent polymer particles, for example typically having an average particle size from 200 µ to 600 µ, or from 300 µ to 500 µ.

According to the present invention, the absorbent core 28 can further comprise an optional cover layer 130, as illustrated in Figure 4, thus providing a layered structure where the layer of absorbent polymer material 110, the non water soluble thermoplastic material 120 and the microspheres 150 are sandwiched between the substrate layer 100 and the cover layer 130.

Also, the substrate layer 100 can be typically provided by a fibrous material, as will be explained in detail below; while the optional cover layer 130 may be provided by the same material as the substrate layer 100, or may be provided by a different material. Suitable materials for the cover layer are for example nonwoven materials, as will be better explained further on.

In general, the layer of absorbent polymer material 110 is stabilized, and typically bonded to the substrate layer 100, by the non water soluble thermoplastic material 120. According to the present invention, the non water soluble thermoplastic material can for example comprise particles of a polymer, such as polyethylene, which upon suitable thermal treatment melt and create bonds within the absorbent polymer material 110, for example provided by absorbent polymer particles, and between the absorbent polymer material 110 and the substrate layer 100 and, if present, the optional cover layer 130.

According to the present invention, the non water soluble thermoplastic material 120 can be an adhesive, typically a hot melt adhesive, which can be also provided as a layer, as illustrated in Figures 3 and 4. In general, according to the present invention, the thermoplastic material, typically a hotmelt adhesive, can be present in the form of fibres, being provided with known means, i.e. the hot melt adhesive can be fiberized.

While thin when dry, the absorbent core typically expands when wetted to provide a high, tenacious fluid-holding ability, and can also avoid collapse when wet. Stability in wet state is also due to the thermoplastic material , e.g. an adhesive, being non water soluble, hence with a bonding capacity non influenced by the presence of water based fluids.

According to an exemplary embodiment of the present invention illustrated in Figure 3, the substrate layer 100 comprises a first surface and a second surface. Conventionally, in the sectional views illustrated in the attached drawings the first surface of each layer can be said to correspond to the top surface, in turn the wearer facing surface of the article 20, while the second surface corresponds to the bottom surface, in turn the garment facing surface. At least portions of the first surface of the substrate layer 100 are in contact with a layer of absorbent polymer material 110. This layer of absorbent polymer material 110 can be typically a non uniform layer, and comprises a first surface and a second surface, wherein by "non uniform" it is meant that the absorbent polymer material 110 is distributed over the substrate layer 100 with non uniform basis weight. Conversely, the second surface of the non uniform layer of absorbent polymer material 110 is in at least partial contact with the first surface of the substrate layer 100. According to an embodiment of the present invention, the non uniform layer of absorbent polymer material 110 can be a discontinuous layer that is a layer typically comprising openings, i.e. areas substantially free of absorbent polymer material, which in certain embodiments can be typically completely surrounded by areas comprising absorbent polymer material, as will be explained in more detail later on. Typically these openings have a diameter or largest span of less than 10 mm, or less than 5 mm, or 3 mm, or 2 mm, or 1.5 mm and of more than 0.5 mm, or 1 mm. At least portions of the second surface of the absorbent polymer material layer 110 are in contact with at least portions of the first surface of the substrate layer material 100. The first surface of the absorbent polymer material 110 defines a certain height of the layer of absorbent polymer material above the first surface of the layer of substrate material 100. When the absorbent polymer material layer 110 is provided as a non uniform layer, typically for example as a discontinuous layer, at least some portions of the first surface of the substrate layer 100 are not covered by absorbent polymer material 110. The layer of absorbent polymer material 110 further comprises the non water soluble thermoplastic material 120 as a layer having a first surface and a second surface. The non water soluble thermoplastic material 120 can be an adhesive 120, typically for example a hot melt adhesive. This layer of non water soluble thermoplastic material 120 serves to at least partially immobilize the absorbent polymer material 110.

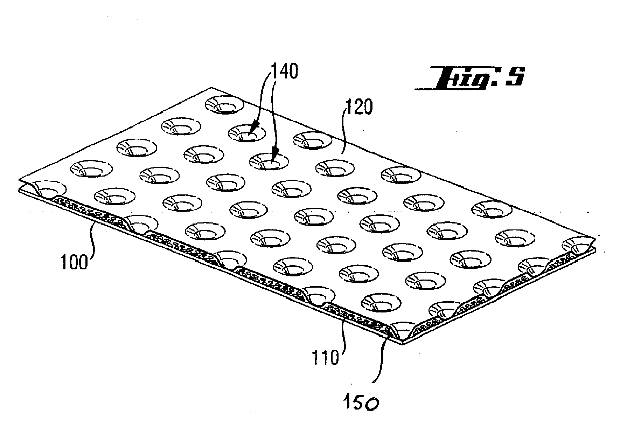

In an embodiment of the present invention the non water soluble thermoplastic material 120, for example a layer of adhesive 120, can be provided as a layer which is partially in contact with the absorbent polymer material 110 and partially in contact with the substrate layer 100. Figures 3 and 5 show such a structure in an exemplary embodiment of the present invention. In this structure the absorbent polymer material layer 110 is provided as a discontinuous layer, a layer of non water soluble thermoplastic material 120 is laid down onto the layer of absorbent polymeric material 110, such that the layer of non water soluble thermoplastic material 120, typically its second surface, is in direct contact with the first surface of the layer of absorbent polymer material 110, but also in direct contact with the first surface of the substrate layer 100, where the substrate layer is not covered by the absorbent polymeric material 110, i.e. typically in correspondence of the openings of the discontinuous layer of absorbent polymer material 110. By "direct contact", as well as more in general "in contact", as used herein, it is meant that there is no further intermediate component layer between e.g. the layer of non water soluble thermoplastic material 120 and the other respective layer in direct contact thereto, such as for example a further fibrous layer. It is however not excluded that a further adhesive material can be comprised between the layer of non water soluble thermoplastic material 120 and the optional cover layer 130, when present, as shown in Figure 4, or the layer of absorbent polymer material 110 or, more typically, between the layer of absorbent polymer material 110 and the substrate layer 100, such as for example a supplementary adhesive material provided onto the first surface of the substrate layer 100 to further stabilize the overlying absorbent polymer material 110. "In direct contact" and "in contact" can hence be considered to comprise in this context a direct, typically adhesive, contact between the layer of non water soluble thermoplastic material 120 and another respective layer as explained above, or more in general direct and, typically, adhesive contact between two layers, e.g. the layer of absorbent polymer material and the substrate layer. This imparts an essentially three-dimensional structure to the layer of non water soluble thermoplastic material 120 which in itself is essentially a two-dimensional structure of relatively small thickness (in z-direction), as compared to the extension in x- and y-direction. In other words, the layer of non water soluble thermoplastic material 120 undulates between the first surface of the absorbent polymer material 110 and the first surface of the substrate layer 100. The areas where the layer of non water soluble thermoplastic material 120 is in contact with the substrate layer 100 are the areas of junction 140.

Thereby, in such an embodiment the layer of non water soluble thermoplastic material 120 can provide spaces to hold the absorbent polymer material 110 typically towards the substrate layer 100, and can thereby immobilize this material. In a further aspect, the layer of non water soluble thermoplastic material 120 bonds to the substrate 100 and thus affixes the absorbent polymer material 110 to the substrate 100.

In the alternative embodiment representatively illustrated in Figure 4 the absorbent core 28 further comprises the optional cover layer 130 having a first surface and a second surface, with the second surface of the cover layer 130 in direct contact with the first surface of the layer of non water soluble thermoplastic material 120.

In the embodiment of Figure 4 portions of the cover layer 130 bond to portions of the substrate layer 100 via the layer of adhesive 120. Thereby, the substrate layer 100 together with the cover layer 130 provides spaces to immobilize the absorbent polymer material 110.

Of course, while the adhesive materials disclosed herein can provide a much improved wet immobilisation, i.e. immobilisation of absorbent polymer material when the article is wet or at least partially loaded, being non water soluble, as explained above, these adhesive materials can also provide a very good immobilisation of absorbent polymer material when the article is dry.

In accordance with the present invention, the absorbent polymer material 110 may also be optionally mixed with fibrous material, which can provide a matrix for further immobilization of the absorbent polymer material. However, typically a relatively low amount of fibrous material can be used, for example less than 40 weight %, less than 20 weight %, or less than 10 weight % of the total weight of the absorbent polymer material 110, positioned within the areas of absorbent polymer material.

According to the present invention, in a typically discontinuous layer of absorbent polymer material 110 the areas of absorbent polymer material can be connected to one another, while the areas of junction 140 can be areas, which in an embodiment may correspond to the openings in the discontinuous layer of absorbent polymer material 110, as shown for example in Figure 5. The areas of absorbent polymer material are then referred to as connected areas. In an alternative embodiment, the areas of junction 140 can be connected to one another. Then, the absorbent polymer material can be deposited in a discrete pattern, or in other words the absorbent polymer material represents islands in a sea of non water soluble thermoplastic material 120. Hence, in summary, a discontinuous layer of absorbent polymer material 110 may comprise connected areas of absorbent polymer material 110, as e.g. illustrated in Figure 5, or may alternatively comprise discrete areas of absorbent polymer material 110.

The present invention, and specifically the embodiments described with reference to Figures 3, 4 and 5 can be used to provide a storage layer of an absorbent core. However, they can also be used to provide the full absorbent core 28 as illustrated in Figure 1. In that case, no further materials wrapping the core, such as for example a top layer and a bottom layer are being used. With reference to the embodiments of Figure 4 the optional cover layer 130 may provide the function of a top layer and the substrate layer 100 may provide the function of a bottom layer of an absorbent core, wherein top and bottom layers respectively can correspond to the body facing and garment facing surfaces of the core 28.

With reference to Figures 3, 4 and 5 the areas of direct contact between the layer of non water soluble thermoplastic material 120 and the substrate layer 100 are referred to as areas of junction 140. The shape, number and disposition of the areas of junction 140 will influence the immobilization of the absorbent polymer material 110. The areas of junction can be for example of squared, rectangular or circular shape. Areas of junction of circular shape can have a diameter of more than 0.5 mm, or more than 1 mm, and of less than 10 mm, or less than 5 mm, or less than 3 mm, or less than 2 mm, or less than 1.5 mm. If the areas of junction 140 are not of circular shape, they can be of a size as to fit inside a circle of any of the diameters given above.

The areas of junction 140 can be disposed in a regular or irregular pattern. For example, the areas of junction 140 may be disposed along lines as shown in Figure 5. These lines may be aligned with the longitudinal axis of the absorbent core, or alternatively they may have a certain angle in respect to the longitudinal edges of the core. A disposition along lines parallel with the longitudinal edges of the absorbent core 28 might create channels in the longitudinal direction which can lead to a lesser wet immobilization, hence for example the areas of junction 140 can be arranged along lines which form an angle of 20 degrees, or 30 degrees, or 40 degrees, or 45 degrees with the longitudinal edges of the absorbent core 28. Another pattern for the areas of junction 140 can be a pattern comprising polygons, for example pentagons and hexagons or a combination of pentagons and hexagons. Also typical can be irregular patterns of areas of junction 140, which also can give a good wet immobilization. Irregular patterns of areas of junction 140 can also give a better fluid handling behaviour in case of absorption of menses or blood or vaginal discharges, since fluid can start diffusing in whichever direction from any initial acquisition point with substantially the same probability of contacting the absorbent polymer material in the e.g. discontinuous layer. Conversely, regular patterns might create preferential paths the fluid could follow with lesser probability of actually contacting the absorbent polymer material.

According to the present invention the adhesive constituting a typical material for the non water soluble thermoplastic material 120, typically e.g. provided as a layer, can comprise any adhesive material, and typically thermoplastic adhesive materials, also referred to as hot melt adhesive material. A variety of thermoplastic adhesive materials can be suitable to immobilize the absorbent polymer material. Some initially thermoplastic adhesive materials may later lose their thermoplasticity due to a curing step, e.g. initiated via heat, UV radiation, electron beam exposure or moisture or other means of curing, leading to the irreversible formation of a crosslinked network of covalent bonds. Those materials having lost their initial thermoplastic behaviour can be herein also understood as non water soluble thermoplastic materials 120.

Without wishing to be bound by theory it has been found that those non waters soluble thermoplastic materials, i.e. typically the thermoplastic or hot melt adhesives, can be most useful for immobilizing the absorbent polymer material 110, which combine good cohesion and good adhesion behaviour. Good adhesion is necessary to ensure that the typically adhesive layer 120 maintains good contact with the absorbent polymer material 110 and in particular with the substrate material 100. Good adhesion is a challenge, namely when a non-woven substrate material is used. Good cohesion ensures that the adhesive does not break, in particular in response to external forces, and namely in response to strain. The adhesive is subject to external forces when the absorbent product has acquired liquid, which is then stored in the absorbent polymer material 110 which in response swells. An exemplary adhesive should allow for such swelling, without breaking and without imparting too many compressive forces, which would restrain the absorbent polymer material 110 from swelling. It may be desirable that the adhesive not break, which would deteriorate the wet immobilization. Exemplary suitable adhesive materials can be as described in patent application EP 1447067, particularly at sections [0050] to [0063].

In general, according to the present invention, the non water soluble thermoplastic material, typically an adhesive material, typically a hotmelt adhesive, can be present in the form of fibres throughout the core, being provided with known means, i.e. the adhesive can be fiberized. Typically, the fibres can have an average thickness of 1 to 100 micrometer and an average length of 5 mm to 50 cm. In particular the layer of adhesive material, typically e.g. a hot melt adhesive, can be provided such as to comprise a net-like structure.

To improve the adhesiveness of the non water soluble thermoplastic material 120 to the substrate layer 100 or to any other layer, in particular any other non-woven layer, such layers may be pretreated with an auxiliary adhesive.

In particular, typical parameters of a hot melt adhesive in accordance with the present invention can be as follows.

In an aspect, the loss angle tan Delta of the adhesive at 60°C should be below the value of 1, or below the value of 0.5. The loss angle tan Delta at 60°C is correlated with the liquid character of an adhesive at elevated ambient temperatures. The lower tan Delta, the more an adhesive behaves like a solid rather than a liquid, i.e. the lower its tendency to flow or to migrate and the lower the tendency of an adhesive superstructure as described herein to deteriorate or even to collapse over time. This value is hence particularly important if the absorbent article is used in a hot climate.

In a further aspect, hot melt adhesives in accordance with the present invention may have a sufficient cohesive strength parameter y. The cohesive strength parameter γ is measured using the Rheological Creep Test as referred to hereinafter. A sufficiently low cohesive strength parameter γ is representative of elastic adhesive which, for example, can be stretched without tearing. If a stress of τ = 1000 Pa is applied, the cohesive strength parameter γ can be less than 100%, less than 90%, or less than 75%. For a stress of τ = 125000 Pa, the cohesive strength parameter γ can be less than 1200%, less than 1000%, or less than 800%.

In the absorbent core 28 of the present invention the substrate layer 100 and the optional cover layer 130 can be typically provided from nonwoven materials, for example spunbonded or carded nonwoven materials, or also airlaid materials, such as for example latex and/or thermal bonded airlaid materials.

Exemplary materials for the substrate layer 100 can comprise fibrous materials comprising cellulose fibres, typically not more than 60% by weight of cellulose fibres, or from 30% to 50% by weight of cellulose fibres. Examples of fibrous materials for the substrate layer 100 can be nonwoven materials, such as for example carded nonwovens, or more typically airlaid or wetlaid fibrous materials, such as for example latex or thermal bonded airlaid fibrous materials, comprising synthetic and natural fibres, such as for example cellulose fibres. Basis weights for the materials of the substrate layer 100 can typically range from 10 g/m² to 120 g/m², or from 40 g/m² to 100 g/m², or also from 50 g/m² to 80 g/m².

Exemplary materials for the optional cover layer 130 can be provided by nonwoven materials comprising synthetic fibres, such as polyethylene (PE), polyethylene terephthalate (PET), polypropylene (PP). As the polymers used for nonwoven production are inherently hydrophobic, they can be typically coated with hydrophilic coatings, for example with durably hydrophilic coatings to provide permanently hydrophilic nonwovens. Other nonwoven materials for the optional cover layer 130 can comprise composite structures such as a so called SMS material, comprising a spunbonded, a melt-blown and a further spunbonded layer. Basis weights for the materials of the cover layer 130 can typically range from 5 g/m² to 80 g/m², or from 10 g/m² to 60 g/m², or also from 20 g/m² to 40 g/m².

According to the present invention the absorbent polymer material 110 in the absorbent core 28 is present throughout the area of the absorbent core in an average basis weight of less than 250 g/m², or of less than 220 g/m², or from 60 g/m² to 180 g/m², or from 100 g/m² to 160 g/m². An average basis weight is typically based on the whole area of the zone of application, i.e. interested by the layer of absorbent polymer material, and hence comprising possible openings included in an e.g. discontinuous layer. Typically, the absorbent polymer material 110 can constitute at least 45%, or at least 50%, or at least 55%, by weight of the absorbent core, wherein the absorbent core can typically correspond to the embodiments described with reference to e.g. Figures 3, 4, and 5, hence generally comprising the substrate layer, the layer of absorbent polymer material, the layer of adhesive, the optional cover layer if present, and any other material possibly comprised within this structure, such as for example the additional fibrous material mentioned above, additional adhesive material, but excluding the microspheres.

Typically the absorbent polymer material for the absorbent cores according to the present invention can comprise absorbent polymer particles having a selected average particle size.

According to the present invention, the layer of absorbent polymer material 110, such as illustrated in the attached drawings, further comprises microspheres 150.

In this application "microspheres" are intended to mean small spherical particles having a flexible, thermoplastic shell and a core that includes a fluid expandable by heating, typically a liquid hydrocarbon. If subjected to suitable heating, the shell of the microspheres softens, the fluid contained therein changes to gaseous state thereby causing expansion of the microsphere up to tens of times the initial volume thereof, typically for example up to more than 40 times.

Typically the microspheres are available from the supplier in the non-expanded state, or in an already expanded state provided by suitable heat treatment.

A known type of microspheres, suitable for this invention, are made under the name of Expancel®, manufactured by Akzo Nobel, that are small spherical particles and have a polymer shell encapsulating a gas. Particularly suitable forms of microspheres that can be used are EXPANCEL® DE (Akzo Nobel) products series and EXPANCEL® DU (Akzo Nobel) products series.

Another example of suitable microspheres includes DUALITE® expanded spheres manufactured by Henkel, which consist of a flexible, thermoplastic hollow microsphere with a core filled with a gas.

According to the present invention, the microspheres 150 can be typically provided in a process step directly to the layer of absorbent polymer material, for example intermixed with it in the same lay-down system.

In general, according to the present invention, the absorbent core 28 can comprise the microspheres 150 in an already expanded state, or in a non-expanded state, or can comprise a combination of expanded and non-expanded microspheres. Typically, the microspheres 150 can be included in an already expanded state.

The amount of microspheres can be selected based upon the desired properties of the core of the disposable product. In general the amount of the microspheres according to the present invention can constitute from 0.5% to 100%, or from 5% to 80%, or also from 10% to 60% by weight of the amount of absorbent polymer material.

According to the present invention the microspheres, owing to their resilient nature, can mitigate the roughness or grittiness feeling that relatively high amounts of absorbent polymer material, typically in particulate form and concentrated in a thin absorbent core, can provide to the user, particularly in the dry state.

The microspheres, particularly in the expanded state, are highly resilient and easy to compress, hence following fluid absorption, and upon swelling of the absorbent polymer material, can deform and accommodate the swelling of the absorbent polymer material, thus providing additional volume for further fluid acquisition and absorption, and ultimately improving the fluid handling capacity of the absorbent core of the present invention.

According to the present invention, the absorbent core can provide a more effective softness and comfort in dry stage as well as a more efficient fluid management, in terms of acquisition, immobilization and absorption, as explained above, which can be particularly useful in case of complex body fluids such as menses or blood. Overall, this increased efficiency in the composite structure according to the present invention can translate in a more effective exploitation of the properties of the absorbent polymer material, also in presence of problematic body fluids such as menses or blood or vaginal discharges, and possibly also in a more efficient use of the entire structure of the absorbent core.

This is achieved in a structure which is typically thin and is capable of employing more completely the absorption and immobilization capacity of the different materials, particularly the absorbent polymer material which can hence provide a particularly thin structure having improved performance during absorption and increased comfort during use.

According to the present invention the absorbent polymer material can be selected among the polyacrylate based polymers described in the PCT Patent Application WO 07/047598, which are polyacrylate based materials very slightly crosslinked, or substantially not crosslinked at all, this further improving the above mentioned synergistic effect. Particularly, said polyacrylate based materials can have an extractable fraction of at least about 30% by weight, between 30% and 80% by weight, or between 32% and 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. The absorbent polymer material can also be selected among the polyacrylate based polymers described in the PCT Patent Application WO 07/046052. Said polymers in fact are particularly effective in absorbing complex body fluids such as menses or blood, and upon absorption of such fluids do not generally show a marked swelling, followed by gel blocking, like traditional superabsorbents, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core. Synergy with microspheres material can be particularly effective with said absorbent polymer materials.

According to the present invention, the absorbent core 28 as described above can fully constitute the absorbent element in an absorbent article, or can constitute part of it, being complemented with other layers in a composite structure. Also, an absorbent article comprising an absorbent core according to the present invention can further comprise a fibrous acquisition layer between the absorbent core 28 and the topsheet. According to an embodiment of the present invention the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from 10 g/m² to 60 g/m², or from 25 g/m² to 40 g/m²_{.}

According to another embodiment of the present invention the absorbent article can comprise a further fibrous layer comprised between the absorbent core 28 and the backsheet, i.e. typically provided at the garment facing surface of the core. This optional layer can be provided by similar fibrous materials as those already described for the substrate layer 100 of the absorbent core of the present invention. This optional fibrous layer according to this further embodiment of the present invention can act as an added wicking layer receiving and distributing excess fluid which might not be fully retained by the absorbent core 28. The presence of cellulose fibres can make the layer particularly effective in acquiring and diffusing the fraction of body fluids like menses or blood which is not completely absorbed by the absorbent polymer material of the absorbent core 28.

### Backsheet

The absorbent article comprising the core according to the present invention can also comprise a backsheet 40. The backsheet primarily has to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

## Claims

1. A layered absorbent core, said core comprising a substrate layer, a layer of absorbent polymer material comprising a non water soluble thermoplastic material,
said layer of absorbent polymer material further comprising microspheres.

2. An absorbent core according to claim 1, wherein said microspheres are in non-expanded form, or in expanded form, or in a mixture of both forms.

3. A layered absorbent core according to any preceding claim, wherein said absorbent polymer material is in particulate form.

4. A layered absorbent core according to any preceding claim, wherein said non water soluble thermoplastic material is in particulate form.

5. A layered absorbent core according to claim 3, wherein said non water soluble thermoplastic material is an adhesive, preferably a hot melt adhesive.

6. A layered absorbent core according to claim 5, wherein said non water soluble thermoplastic material is a fiberized hot melt adhesive.

7. A layered absorbent core according to any preceding claim, wherein said non water soluble thermoplastic material is comprised as a layer.

8. An absorbent core according to any preceding claim, wherein said microspheres constitute from 0.5% to 100%, preferably from 5% to 80%, more preferably from 10% to 60% by weight of the amount of said absorbent polymer material.

9. A layered absorbent core according to any preceding claim, wherein
said substrate layer comprises a first surface and a second surface,
said layer of absorbent polymer material is non uniform and comprises a first surface and a second surface,
said non water soluble thermoplastic material is comprised as a layer comprising a first surface and a second surface,
said second surface of said non uniform layer of absorbent polymer material is in at least partial contact with said first surface of said substrate layer,
portions of said second surface of said layer of adhesive are in direct contact with said first surface of said substrate layer and portions of said second surface of said layer of adhesive are in direct contact with said first surface of said non uniform layer of absorbent polymer material.

10. A layered absorbent core according to claim 9, comprising a cover layer comprising a first surface and a second surface, wherein said second surface of said cover layer is in direct contact with said first surface of said layer of non water soluble thermoplastic material.

11. An absorbent article comprising a liquid permeable topsheet, a backsheet and an absorbent core comprised therebetween, said absorbent core according to any preceding claim.

12. An absorbent article according to claim 11, wherein said article is a sanitary napkin or a pantiliner.
